(19) Europäisches Patentamt
European Patent Office
Office européen des brevets

(11) Numéro de publication: **0 239 508 A2**

(12) **DEMANDE DE BREVET EUROPEEN**

(21) Numéro de dépôt: **87420046.2**

(22) Date de dépôt: **17.02.87**

(51) Int. Cl.⁴: **C 07 D 235/24**, A 01 N 43/52, C 07 D 235/10, C 07 C 87/60

(30) Priorité: **19.02.86 FR 8602455**

(43) Date de publication de la demande: **30.09.87 Bulletin 87/40**

(84) Etats contractants désignés: **AT BE CH DE ES FR GB GR IT LI LU NL SE**

(71) Demandeur: **RHONE-POULENC AGROCHIMIE, 14-20, rue Pierre Baizet, F-69009 Lyon (FR)**

(72) Inventeur: **Gouot, Jean-Marie, 24 Allée des Eglantiers, F-69450 St Cyr au Mont d'Or (FR)**
Inventeur: **Souche, Jean-Luc, 45 Chemin des Fontanières, F-69350 La Mulatière (FR)**

(74) Mandataire: **Ranguis, Patrick Frédéric et al, RHONE POULENC AGROCHIMIE Service DPI B.P. 9163, F-69263 Lyon Cédex 09 (FR)**

(54) **Nouveaux dérivés du cyano-2 benzimidazole, leur préparation et leur utilisation comme fongicide, leur association avec d'autres fongicides.**

(57) L'invention concerne de nouveaux dérivés du cyano-2 benzimidazole.

Ces composés répondent à la formule

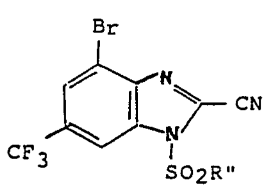

(I)

dans laquelle R″ représente un radical dialkylamino comportant de 2 à 4 atomes de carbone.

Ces composés présentent une persistance d'efficacité antifongique améliorée. Ils sont utilisables pour la lutte contre les champignons phytopathogénes.

L'invention concerne également les associations de ces dérivés avec d'autres fongicides.

EP 0 239 508 A2

La présente invention concerne de nouveaux dérivés du cyano-2 benzimidazole et les associations de ces produits avec certains fongicides. L'invention concerne également des procédés de préparation desdits produits et l'utilisation pour la protection des cultures contre les maladies fongiques notamment des mildious desdits produits et desdites associations. Elle concerne de plus les composés utilisables pour la préparation de ces nouveaux dérivés du cyano-2 benzimidazole.

On sait que les maladies provoquées par les champignons de type oomycètes sont particulièrement graves. Parmi le nombre important de maladies foliaires ainsi provoquées on peut citer les mildious de la vigne, du tabac, des tomates, des pommes de terre et des cucurbitacées.

Il a déjà été proposé par EP-A-0087375 de nouveaux dérivés du 2-cyano benzimidazole utilisables dans des compositions fongicides à usage agricole notamment en tant que fongicide de contact et présentant une bonne efficacité vis à vis de plusieurs types de mildiou, notamment le mildiou des solanacées (par exemple de la tomate et de la pomme de terre), Phytophthora infestans, le mildiou du tabac, Peronospora tabacina et Phytophthora parasitica, le mildiou de la vigne Plasmopara viticola, le mildiou des cucurbitacées Pseudoperonospora cubensis.

Il est néanmoins souhaitable de toujours élargir le spectre d'activité et les performances d'un fongicide.

Un but de la présente invention est de fournir des produits et associations ayant une activité antifongique améliorée notamment vis a vis des mildious.

Un autre but de la présente invention est de fournir des produits et associations ayant une toxicité réduite.

Un autre but de la présente invention est de fournir des dérivés du cyano-benzimidazole qui présentent une persistance d'efficacité antifongique supérieure à

celle des composés homologues les plus proches décrits dans la demande de brevet européen 0087 375.

Par "persistance de l'efficacité fongique", on entend, la durée pendant laquelle l'efficacité antifongique d'un composé continue à se maintenir à un niveau satisfaisant après un traitement antifongique effectué sur une culture au moyen de ce composé.

On estime généralement que la protection d'une culture contre les maladies fongiques reste à un niveau satisfaisant tant que le coefficient d'efficacité antifongique reste au moins égal à 80 %. Cette protection est jugée tout à fait excellente lorsque ce coefficient est au moins égal égal à 90 %.

En pratique, la protection des cultures contre les maladies fongiques s'effectue généralement en appliquant sur ces cultures une succession de traitements antifongiques à intervalles déterminés, pendant toute la période de croissance de la végétation, en utilisant pour cela soit une seule matière active antifongique, soit une association de plusieurs matières actives antifongiques.

L'utilisation pour ces traitements d'un composé antifongique seul ou en association présentant une persistance d'efficacité antifongique améliorée est particulièrement avantageuse, car elle permet d'envisager une diminution de la cadence des traitements et par ailleurs, d'assurer aux cultures ou plantes traitées une protection plus durable pendant la période allant du moment où est effectué le dernier traitement antifongique de la série et le moment de la récolte.

Un but de l'invention est donc de mettre à la disposition de l'agriculture un produit antifongique éventuellement en association, actif vis-à-vis de différents types de mildious, notamment les mildious de la pomme de terre, de la vigne, de la tomate, du concombre et du tabac et présentant à la fois une excellente efficacité antifongique immédiate et une persistance d'efficacité

antifongique améliorée.

Il a maintenant été trouvé que ces buts pouvaient être atteints, d'une part grâce aux produits de l'invention.

Ces produits sont des dérivés de cyano-2 benzimidazole et répondent à la formule (I) : dans laquelle R" représente un radical dialkylamino comportant de 2 à 4 atomes de carbone, de préférence le radical diméthylamino.

La formule (I) ainsi que les formules mentionnées ci-après sont réunies en fin de description.

Il a également été trouvé que les buts précités pouvaient être atteints grâce à l'association d'un ou plusieurs composés de formule (I) avec au moins un fongicide du groupe (II) c'est-à-dire un fongicide choisi dans les sous-classes suivantes :

1. Les dérivés chlorés ou nitrés du benzène comme le quintozène ou le chlorothalonil,
2. Les dérivés dicarboximides et imides cycliques comme le captane, le folpel, le captafol, l'iprodione, la procymidone, la vinclozoline, le fluoroimide, le chlozolinate, le metomeclan,
3. Les dérivés comprenant un ou plusieurs hétérocycles comme les quinoléines (éthoxyquine), les morpholines (dodémorphe, tridémorphe, fenpropimorphe), les pipéridines (fenpropidine), les piperazines (triforine),
4. Les dérivés de l'acide dithiocarbamique comme le manèbe, le mancozèbe , le thirame, le metiram ou le zinèbe ou le propinèbe.
5. Les dérivés du phénol comme le dinocap ou le binapacryl,
6. Les dérivés des quinones comme le dithianon, le chloranil,
7. Les dérivés de l'acide carbamique et des benzimidazoles comme le carbendazime, le bénomyl, le thiophanate-méthyl, thiabendazole, le fubéridazol.
8. Les dérivé soufrés comme le dazomet ou l'étridiazole ou le soufre, le fenaminosulf,

9. Les diazines ou triazines telles que le chinométhionate, le fénarimol, l'anilazine, le nuarimol, le bupirimat, l'éthylrimol, le pyrazophos, la diclomezine,

10. Les sulfamides telles que le dichlofluanide, le tolylfluanide,

11. Les guanidines telles que la doguadine,

12. Les triazoles tels que par exemple ceux décrits dans le brevet britannique n°2046260 dont le contenu est incorporé par référence comme le diniconazole ou d'autres triazoles connus, le propiconazole, le triadimefon, le triadimenol, le diclobutrazol, le bitertanol, le penconazol, l'hexaconazole, le flusilazole, le myclobutanil, le SAN 619F (Sandoz), l'ethyltrianól, le fluotrimazole, le flutriafol, les triazoles tels que ceux décrits dans la demande de brevet européen n°0151084, le triazole denommé : 2-(2,4-dichloro 1-phényl) 2-méthyl (1,2,4-triazol 1-yl) 5-trifluoroéthoxy tétrahydrofuranne ou ses formes salifiées. Celui-ci est préparé comme indiqué ci aprés.

Selon un procédé décrit dans la demande de brevet européen n°0151084, on mélange 40 ml de trifluoroéthanol avec 10,7 g de produit de formule (XI) préparé selon l'exemple 2 de la demande de brevet européen n°0151084. On fait barboter du HCl gazeux de manière à avoir une augmentation pondérale de 1,39 g. On chauffe 2 heures à 70°C. On verse dans 500 ml d'eau contenant 10 g de $Na_2CO_3$. On extrait à l'acétate d'éthyle ; la solution organique est lavée à l'eau, sèchée, filtrée, évaporée. On obtient ainsi 8 g de cristaux constitués d'un mélange de 2 diastéréoisomères du produit de formule (I), fondant à partir de 80°C et présentant en RMN un déplacement protonique delta à 5,18 et 5,41 ppm.

13. Les imidazoles comme le prochloraz ou l'imazalil, le triflumizole,

14. Le cuivre ou les dérivés organiques ou inorganiques du cuivre comme le cuprosan.

15. Les organo stanniques comme le fentine acétate.
16. Les anilazines.
17. Les phosphites d'amines décrits dans le brevet belge n°890 114, dont le contenu est incorporé par référence.
18. Les dérivés de la pyridine tels que le buthiobate, le pyrifenox.
19. Les dérivés de l'amino-2 pyridine tels que le fluazinam.
20. Les dérivés de l'isoxazole et de l'isoxazolone tels que l'hymexazole et le drazoxolon.
21. Les dérivés des phényl-carbamates tels que le diethofencarb (isopropyl 3,4-diethoxyphenyl carbamate), le methyl N-(3,5-dichlorophenyl) carbamate, et les autres produits actifs sur les souches de champignons résistantes aux benzimidazoles (par exemple : Botrytis cinerea).

Les matières actives du groupe (II) sont des matières actives connues, la plupart d'entre elles étant décrites en détail dans des ouvrages tels que "The Pesticidal Manual" édité par "The British Crop Protection Council" dont la 7ème édition date de 1983.

Les noms chimiques des produits énumérés ci-dessus sont indiqués au tableau annexé selon la nomenclature anglo-saxonne, c'est-à-dire avec l'indication de la position des substituants placée avant celui-ci.

Les associations selon l'invention sont le plus souvent de type binaire (une seule matière active du groupe (II) mais on utilise aussi quelquefois des associations ternaires (2 matières actives du groupe II) ou quaternaire (3 matières actives du groupe II).

Parmi les matières actives, on préfèrera celles choisies parmi les sous-classes 1, 2, 4, 14, 15.

Parmi les matières actives du groupe II, on préfèrera encore les matières actives suivantes : chlorothalonil, iprodione, fentine-acétate, dinocap, manèbe, mancozèbe, zinèbe, captane, captafol, folpel, procymidone, le cuivre et les dérivés du cuivre tels que l'oxychlorure de cuivre ou quinoléate de cuivre, en association binaire,

ternaire ou quaternaire.

Il est également possible d'associer aux associations précitées un composé constitué par :

a) l'acide phosphoreux ou ses sels alcalins ou les dérivés de l'acide phosphoreux de préférence les dérivés de formule (II) dans laquelle :

R' est un atome d'hydrogène ou de métal alcalin ou un radical alkyle contenant de 2 à 4 atomes de carbone ;

M est un atome d'hydrogène ou un atome d'un métal alcalin, alcalino terreux ou d'aluminium ;

n' est un nombre entier de 1 à 3 égal à la valence M.

De préférence, lorsque R' est un atome d'hydrogène ou de métal alcalin, M est un métal alcalin et n'=1.

De préférence, le dérivé est un alcoyl-phosphite métallique, de préférence le phosétyl-Al ou un dérivé alcalin de l'acide phosphoreux ou l'acide phosphoreux lui-même.

b) les acétamides tels que ceux décrits dans le brevet US n° 3,957,847 dont le contenu est incorporé par référence et de préférence répondant à la formule (III) dans laquelle : $R_1$, $R_2$ identiques ou différents représentent un groupement alkyle de $C_1$ à $C_{13}$ de préférence de $C_1$ à $C_4$ et de préférence le cymoxanil ($R_1 = CH_3$, $R_2 = C_2H_5$).

c) Les acylanilides tels que ceux décrits dans les brevets belges n° 827 671 et 827 419 ou européen n° 0026873 dont le contenu est incorporé pr référence et de préférence :

1) ceux de formule (IV) dans laquelle :

$R_3$, $R_4$ identiques ou différents représentent un atome d'hydrogène ou un groupe alkyle de $C_1$ à $C_4$, de préférence le méthyle, ou alcoxy de $C_1$ à $C_4$, avantageusement $R_3 = R_4 =$ méthyle.

$R_5$ représente un groupe $-CH_2-R_6$ dans lequel $R_6$ est un groupement alcoxy de $C_1$ à $C_4$, de préférence méthoxy, ou aryle de préférence phényle,

un atome d'halogène de préférence le chlore, ou $R_5$ représente un cycloalkyle de $C_3$ à $C_6$ de préférence le cyclopropane, ou un hétérocycle en $C_5$ ou $C_6$, de préférence $C_5$, comportant un ou deux hétéroatomes de préférence le 2-furyle, le 5-isoxazolyle, le 3-oxazolyle.

$R_7$ est un atome d'hydrogène, d'halogène ou un radical alkyle de $C_2$ à $C_4$, de préférence méthyle et avantageusement, le métalaxyl, le furalaxyl, le bénalaxyl, le composé où $R_3=R_4=CH_3$, $R_7$ = H, $R_5$ = 5-isoxazolyle (LAB 149 202 F).

2) ou ceux de formule (V) dans laquelle :

$R_3$, $R_4$, $R_5$, $R_6$, $R_7$ ont la même signification que pour la formule (IV) avec les mêmes variantes préférées et $R_8$ est un cycle 3-oxazolidinonyl ou 3-butyrolactonyl ou le 3-thiobutyrolactonyl ou 3-thiooxazolidinonyl, avantageusement l'oxadixyl, le cyprofuram, l'ofurace, le CGA 68099 (RE 26745) dans lequel $R_3$ = $R_4$ = méthyle, $R_7$ = H, $R_5$ = $-CH_2OCH_3$, $R_8$ = 3-butyrolactonyl, le RE 26940 où $R_3$ = $R_4$ = $CH_3$, $R_7$ = H, $R_5$ = $CH_2OCH_3$, $R_8$ = 3-thiobutyrolactonyl, le M 12279 ou $R_3$ = $R_4$ = $CH_3$, $R_5$ = $-CH_2OCH_3$, $R_7$ = 3-Cl, $R_8$ = 3-oxazolidinonyl.

Certains de ces composés sont décrits dans le brevet belge n° 884 661 dont le contenu est incorporé par référence. Les composés de formule (I) sont décrits dans la demande de brevet français non publiée déposée le 19 février 1986 sous le numéro d'enregistrement 8602455. Ces composés sont donc inclus dans le groupe (II). Comme association, on peut citer par exemple le Mikal (Phoséthyl-Al et Folpel) associé à un produit de l'invention et le Rhodax (Phosethyl-Al+ mancozebe).

L'invention concerne, également, un procédé de préparation des composés de formule I caractérisé en ce qu'il consiste à faire réagir le cyano-2 bromo-4 trifluorométhyl-6 benzimidazole de formule (VI) ou l'un de

ses sels, avantageusement un sel de métal alcalin, ou alcalino-terreux, ou d'ammonium, éventuellement substitué, avec l'halogénure de formule (VII) X - $SO_2$- R"dans laquelle X représente un atome d'halogène (de préférence un atome de chlore) et R" a la même signification que dans la formule (I).

La réaction du composé (VI) sur l'halogènure (VII) s'effectue avantageusement en présence d'un accepteur d'acide, en milieu anhydre ou non anhydre, dans un solvant, inerte dans les conditions de la réaction, généralement à la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine. Comme solvants on utilise avantageusement des solvants aprotiques polaires, tels que par exemple, le diméthylformamide, le diméthylacétamide, le diméthylsulfoxyde, l'acétone, la méthyléthylcétone, l'acétonitrile, la N-méthylpyrrolidone, l'acétate d'éthyle. Si désiré, cette réaction peut être effectuée en présence d'un catalyseur approprié. Comme catalyseur utilisable, on peut mentionner des catalyseurs de transfert de phases, tels que, par exemple, des sels d'ammonium quaternaire.

La réaction du sel (de métal alcalin, ou alcalino-terreux, ou d'ammonium, éventuellement substitué) du composé (VI) sur l'halogénure (VII) ne nécessite pas la présence d'un accepteur d'acide. Elle s'effectue en milieu anhydre ou non anhydre, dans un solvant, inerte dans les conditions de la réaction, généralement à la température d'ébullition du solvant. Les solvants aprotiques polaires cités plus haut peuvent être utilisés avantageusement dans cette réaction.

Si désiré, cette réaction peut être effectuée en présence d'un catalyseur approprié, tel que, par exemple, un catalyseur de transfert de phases (par exemple un dérivé d'ammonium quaternaire).

Le sel de métal alcalin, ou alcalino-terreux, ou d'ammonium, éventuellement substitué, du composé (VI) est préparé dans une opération préalable, éventuellement effectuée in situ, par action d'une base appropriée (par exemple soude, potasse, ammoniaque, baryte, chaux, etc..) ou d'un carbonate de métal alcalin, ou d'un alcoolate de métal alcalin (par exemple méthylate de sodium ou éthylate de sodium ou potassium) sur ce composé (VI).

En fin de réaction, quelque soit le procédé utilisé, le composé formé est isolé du milieu réactionnel par tout moyen en soi connu tel que, par exemple par distillation du solvant, ou par cristallisation du produit dans le milieu réactionnel, ou par filtration, puis, si nécessaire, ce composé est purifié par des méthodes usuelles telles que recristallisation dans un solvant approprié.

Le cyano-2 bromo-4 trifluorométhyl-6 benzimidazole, de formule (VI) peut être préparé par action de l'ammoniaque sur le trichlorométhyl-2 bromo-4 trifluorométhyl-6 benzimidazole, de formule (VIII) selon la méthode décrite dans la demande de brevet européen n° 87 375 (Méthode A) et le brevet américain n° 3 576 818. Pour réaliser cette opération le composé (VIII) est avantageusement dissous dans un solvant tel que le diméthylformamide, le diméthoxy-1,2 éthane, l'acétonitrile, l'acétate d'éthyle ou l'alcool éthylique, cette liste étant non limitative. Il peut également être préparé selon l'une ou l'autre des méthodes B et C décrites dans la demande de brevet européen n° 87 375.

Si désiré, on peut effectuer successivement dans un même réacteur la préparation du composé de formule (VI) à partir du composé de formule (VIII), puis la transformation du composé de formule (VI) en composé de formule (I), sans isoler le composé intermédiairement formé (composé de formule (VI) ou sel de ce composé).

Le composé de formule (VIII) peut être préparé par action du trichloroacétimidate de méthyle sur le diamino-1,2 bromo-3 trifluorométhyl-5 benzène, de formule (IX) ou sur un sel de ce composé de formule (IX) formé avec un acide fort. De préférence ce sel est formé en associant une mole du composé de formule (IX) avec une mole d'un monoacide fort, tel que par exemple HCl ou HBr, ou encore en associant deux moles de ce composé de formule (IX) avec une mole d'un diacide fort, tel que par exemple $H_2SO_4$. De préférence, ce sel est le monochlorhydrate du composé de formule (IX).

La réaction s'effectue avantageusement dès la température ambiante, en milieu acide acétique, ou dans des alcanols inférieurs tels que l'éthanol ou le méthanol. Elle peut également être effectuée à la température de reflux des solvants utilisés.

Le composé de formule (IX) peut être préparé à partir de la bromo-2 trifluorométhyl-4 nitro-6 aniline, de formule (X) par réduction du groupe nitro en groupe amino. Cette réduction peut s'effectuer par tout moyen en soi connu pour permettre la réduction d'un nitrobenzène en aniline correspondante, par exemple en faisant agir le chlorure stanneux en présence d'acide chlorhydrique concentré, cette dernière méthode permettant d'obtenir le monochlorhydrate du composé de formule (IX).

Les dérivés de trichlorométhyl-2 bromo-4 benzimidazole répondant aux formules (VI) et (VIII), ainsi que les sels de ces composés, notamment les sels de métaux alcalins ou d'ammonium, formés par réaction de ces composés avec une base minérale ou organique appropriée sont également compris, en tant que produits nouveaux, dans le cadre de l'invention. Il en est de mêmùe pour les dérivés d'aniline répondant aux formules (IX) et (X).

De préférence R" est le radical diméthylamino.

Les associations selon l'invention sont de préférence et le plus souvent de type binaire (une seule

matière active du groupe (II) mais on utilise aussi des associations ternaires (2 matières actives groupe (II)), par exemple avec un composé de formule II à V, ou quelquefois quaternaire.

On utilisera généralement pour les buts précités un produit dans lequel le rapport en poids du ou des composés du groupe (I) au ou aux composés du groupe (II) est compris entre 1 : 10 000 et 100, de préférence entre 1/1000 et 10.

Ces associations selon l'invention sont destinés à une utilisation simultanée, séparée ou étalée dans le temps en traitement fongicide des végétaux, notamment fongicide antimildiou.

Dans le cas d'une utilisation simultanée, il est avantageux d'utiliser des produits prêts à l'emploi contenant l'association des matières actives décrites plus haut. On peut également utiliser des produits préparés juste avant l'emploi, par mélange extemporaire des matières actives ou des compositions contenant chacune des matières actives comme elles sont décrites ci-après.

L'utilisation peut également être espacée dans le temps.

On peut également utiliser le produit en traitant les cultures à protéger successivement par l'une puis l'autre des matières actives (I) et (II) de manière à former in situ sur la plante le produit selon l'invention.

Les produits selon l'invention sont donc utilisables à titre de fongicides agricoles. Il sont utiles tout particulièrement pour la protection des cultures de solanacées, tabac, vigne, concombres, contre les mildious de ces cultures, (Phytophthora infestans, Peronospora Tabacina, Phytophthora parasitica, Plasmospora viticola, Pseudoperonospora cubensis).

Ils sont tout particulièrement utiles contre les mildious de solanacées (tomates, pommes de terre, tabac) des concombres, de la vigne.

Les produits sont préparés par simple mélange des

composés des groupes (I) et (II) les constituants.

Pour leur emploi pratique, les produits selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un produit selon l'invention tel que décrit précédemment en association avec les supports (ou diluants) solides ou liquides inertes, acceptables en agriculture et/ou les agents tensio-actifs compatibles avec la matière active, également acceptables en agriculture. En particulier sont utilisables les supports inertes et usuels et les agents tensio-actifs usuels.

Ces compositions font également partie de la présente invention. Elles contiennent généralement de 0,5 à 95 % de matière active. Leur teneur en agent tensio actif est en général comprise entre 0 % et 20 % en poids.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, engrais liquides et oligoéléments etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras

ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les produits se trouvent donc généralement sous forme de compositions ; ces compositions selon l'invention sont elles-mêmes sous des formes assez diverses, liquides ou solides.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Comme formes de compositions solides, on peut citer les poudres pour poudrage ou dispersion (à teneur en produit pouvant aller jusqu'à 100 %) et les granulés, notamment ceux obtenus par extrusion, par compactage, par imprégnation d'un support granulé, par granulation à partir d'une poudre (la teneur en produit dans ces granulés étant entre 1 et 80 % pour ces derniers cas).

Les concentrés émulsionnables ou solubles comprennent le plus souvent 10 à 80 % de matière active, les émulsions ou solutions prêtes à l'application contenant, quant à elles, 0,001 à 20 % de matière active.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières

actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Par exemple, en plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 5 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 5 à 95 % de matière active, et elles contiennent habituellement, en plus du support solide, de 0 à 5 % d'un agent mouillant, de 3 à 10 % d'un agent

dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici la composition de quelques concentrés émulsionnables :

Exemple F (formulation) 1

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique q.s.p | 1 litre |

Selon une autre formule de concentré émulsionnable, on utilise :

Exemple F 2 :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

A partir de ces concentrés, on peut obtenir par dilution avec de l'eau des émulsions de toute concentration désirée, qui conviennent particulièrement à l'application sur les feuilles.

A titre d'exemple, voici diverses compositions de poudres mouillables (ou poudres à pulvériser) :

Exemple F 3 :

- matière active ................................ 50 %
- alcool gras éthoxylé (agent mouillant)......... 2,5 %
- phényléthylphénol éthoxylé (agent dispersant).. 5 %
- craie (support inerte) ......................42,5 %

Exemple F 4 :

- matière active ................................ 10 %
- alcool synthétique oxo de type ramifié, en $C_{13}$

éthoxylé par 8 à 10 oxyde d'éthylène (agent mouillant) ....................................0,75 %
- lignosulfonate de calcium neutre (agent dispersant ........................................ 12 %
- carbonate de calcium (charge inerte) ....q.s.p. 100 %

<u>Exemple F 5</u> : cette poudre mouillable contient les mêmes ingrédients que dans l'exemple précédent, dans les proportions ci-après :

- matière active .................................. 75 %
- agent mouillant ...............................1,50 %
- agent dispersant ................................8 %
- carbonate de calcium (charge inerte) ....q.s.p. 100 %

<u>Exemple F 6</u> :

- matière active .................................. 90 %
- alcool gras éthoxylé (agent mouillant ........... 4 %
- phényléthylphénol éthoxylé (agent dispersant) ... 6 %

<u>Exemple F 7</u> :

- matière active .................................. 50 %
- mélange de tensio-actifs anioniques et non ioniques (agent mouillant) ..................... 2,5 %
- lignosulfonate de sodium (agent dispersant) .... 5 %
- argile kaolinique (support inerte) .............42,5 %

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être avantageusement formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par

des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple F 8 : Granulés dispersibles

Dans un mélangeur, on mélange 90 % en poids de matière active (composé n° 1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple F 9 : Granulés dispersibles

Dans un mélangeur, on mélange les constituants suivants :

- matière active (composé n° 1) .................. 75 %
- agent mouillant (alkylnaphtalène sulfonate de sodium ....................................... 2 %
- agent dispersant (polynaphtalène sulfonate de sodium) ...................................... 8 %
- charge inerte insoluble dans l'eau (kaolin) ..... 15 %

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Les préparations liquides, diluées ou concentrées, seules ou associées à d'autres pesticides ou engrais, peuvent être avantageusement distribuées à proximité des plantes par irrigation, notamment au goutte à goutte avec un dispositif approprié et par aspersion.

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids). Leur teneur en agent tensio-actif est avantageusement comprise entre 5 % et 40 % en poids.

Les doses d'emploi dans le cas d'une utilisation comme fongicides des composés ou association selon l'invention peuvent varier dans de larges limites, notamment selon la virulence des champignons et les conditions climatiques.

D'une manière générale des compositions contenant 0,5 à 5000 ppm de substance active conviennent bien ; ces

valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

L'invention concerne également un procédé pour combattre les maladies fongiques caractérisé en ce qu'on applique en une ou plusieurs fois sur ces plantes une quantité efficace d'un produit ou d'une association ou d'une composition selon l'invention.

Dans ce procédé en général le composé du groupe (I) est avantageusement appliqué à raison de 2 g/ha à 250 g/ha (de préférence de 4 g/ha à 220 g/ha) selon les végétaux à traiter.

De préférence, on utilisera dans le cas des solanacées 2 à 200 g/ha de matière active et avantageusement de 4 à 160 g/ha.

De préférence, on utilisera dans le cas du tabac pour Peronospora tabacina 80 à 220 g/ha et pour Phytophthora parasitica 60 à 120 g/ha.

De préférence, on utilisera pour le cas de la vigne 100 à 250 g/ha.

De préférence, on utilisera pour le cas des cucurbitacées 2 à 50 g/ha.

Dans le cas des associations, on utilisera 100 à 3000 g/ha de composés du groupe (II) seuls ou en mélange selon les composés choisis.

En pratique, des doses allant de 5 g/hl à 100 g/hl correspondent à des doses de 50 g/ha à 1000 g/ha environ.

Les exemples ci-après, décrits à titre non limitatif, illustrent la préparation des composés selon l'invention ainsi que leur application à la lutte contre les atteintes fongiques.

La structure des composés décrits dans ce qui suit a été confirmée par spectrométrie de résonance magnétique nucléaire et/ou spectrométrie infrarouge.

Exemple 1 : Préparation du diméthylsulfamoyl-1 cyano-2 bromo-4 trifluorométhyl-6 benzimidazole

A une suspension de 1,5 g (0,024 mole) de potasse en écailles dans 80 ml d'acétone, on ajoute, à température ambiante (20°C environ), 7,0 g (0,024 mole) de bromo-4 cyano-2 trifluorométhyl-6 benzimidazole. On observe la dissolution progressive des réactifs et une élévation de la température qui atteint 30°C. La dissolution est complète au bout d'une heure.

On ajoute alors au mélange réactionnel, en 10 minutes, 2,6 ml de chlorure de diméthylsulfamoyle (0,024 mole) et le mélange réactionnel est agité pendant 18 heures puis versé sur 1 litre d'eau distillée. Le précipité obtenu est essoré sur filtre puis lavé par de l'eau (4 x 50 ml), puis par de l'éthanol (50 ml) et enfin par de l'éther éthylique (100 ml). Après séchage, on obtient ainsi 4,3 g de diméthylsulfamoyl-1 cyano-2 bromo-4 trifluorométhyl-6 benzimidazole (composé A) fondant à 198°C. Rendement 45, 1%.

Le cyano-2 bromo-4 trifluorométhyl-6 benzimidazole est préparé comme indiqué ci-après :

A 430 ml d'ammoniaque à 29 %, on ajoute en 45 minutes, 50,8 g (0,13 mole) de bromo-4 trichlorométhyl-2 trifluorométhyl-6 benzimidazole en solution dans 200 ml d'éthanol.

La température du mélange réactionnel est maintenue au voisinage de 25°C. Après une heure d'agitation à température ambiante, le mélange réactionnel est filtré. On acidifie le filtrat jusqu'à pH 1, puis décante. La phase organique est lavée à l'eau. Cette huile cristallise ; le solide est lavé à l'eau puis séché .

On obtient ainsi 30,23 g de cyano-2 bromo-4 trifluorométhyl-6 benzimidazole fondant à 130°C. Rendement : 78,4 %

Le trichlorométhyl-2 bromo-4 trifluorométhyl-6 benzimidazole est préparé comme indiqué ci-après :

Dans une suspension de 81,1 g (0,28 mole) de monochlorhydrate de diamino-1,2 bromo-3 trifluorométhyl-5 benzène dans 600 ml d'acide acétique, on coule en une demi-heure 54 ml de trichloroacétimidate de méthyle, à 21°C. La température monte jusqu'à 40°C. Au bout de 2 heures, on observe une cristallisation dans le milieu réactionnel que l'on maintient sous agitation à 20°C pendant 18 heures. Le mélange réactionnel est alors versé sur 2 litres d'eau distillée. Le précipité formé est essoré, lavé par de l'eau (2 x 200 ml), puis essoré complètement. Après séchage prolongé, on obtient 101 g de trichlorométhyl-2 bromo-4 trifluorométhyl-6 benzimidazole, fondant à 208°C. Rendement 95 %.

Le diamino-1,2 bromo-3 trifluorométhyl-5 benzène a été préparé, sous forme de monochlorhydrate comme indiqué ci-après :

On ajoute par petites fractions en 1 heure et demie 336 g (1,18 moles) de bromo-2 trifluorométhyl-4 nitro-6 aniline à une solution portée à 40°C, de 840 g de dihydrate de chlorure stanneux dans 2,0 l d'HCl concentré. La température s'élève jusqu'à 80°C. On observe la formation d'un important précipité blanc. Après la fin de l'addition, le mélange est chauffé pendant 1 heure à 80°C, puis refroidi jusqu'à 10°C. Le précipité formé est recueilli sur filtre, lavé par HCl concentré (400 ml) puis avec du dichlorométhane (2 x 500 ml) et enfin séché à l'air pendant 2 jours.

On obtient ainsi 297 g de diamino-1,2 bromo-3 trifluorométhyl-5 benzène, sous la forme de monochlorhydrate, fondant à une température supérieure à 270°C. Rendement 86,3 %.

La bromo-2 trifluorométhyl-4 nitro-6 aniline a été préparée comme indiqué ci-après.

Dans un ballon refroidi, on introduit 900 ml d'eau puis on ajoute 600 ml d'acide sulfurique concentré. On ajoute alors 150 g (0,73 mole) de nitro-2 trifluorométhyl-4

aniline et 117 g (0,73 mole) de brome en 1 h 30. On poursuit l'agitation pendant 18 heures, filtre puis lave à l'eau. Après séchage on obtient 188,5 g de bromo-2 trifluorométhyl-4 nitro-6 aniline fondant à 70°C. Rendement 90,6 %.

Exemple 2 :

On a traité 2 séries de 4 parcelles de terrain de 10 m2 portant chacune une culture de pomme de terre de la variété SIRTEMA infestée par du mildiou (Phytophthora Infestans). Ces plants ont été traités 4 fois à 10 jours d'intervalle à l'aide de 2 bouillies fongicides définies ci-après et répandues à raison de 1000 l/ha et ces bouillies contenaient 15 g/hl de matière active, préparée en diluant par de l'eau une poudre mouillable ayant la composition pondérale suivante :

- matière active (dérivé du cyano-2 benzimidazole) 50 %
- agent mouillant : ATLOX 4853 B (mélange de tensio actifs anioniques et non ioniques) 2,5 %
- agent dispersant (lignosulfonate de sodium) 5 %
- kaolin (support inerte) 42,5 %

Les essais ont été effectués au moyen du composé décrit dans l'exemple 1 ci-dessus (composé A) en prenant comme produit de comparaison le diméthylsulfamoyl-1 cyano-2 dibromo-4,6 benzimidazole décrit comme composé n° 39 dans la demande de brevet européen 87 375.

Les résultats suivants ont été obtenus :

| | | COMPOSE A | COMPOSE N°39 |
|---|---|---|---|
| Nombre de taches sur les folioles portés par dix tiges Mesure effectuée 8 jours après le 2ème traitement | | 7 | 42,3 |
| Nombre de folioles attaqués pour un ensemble de dix tiges Mesure effectuée 11 jours après le 3ème traitement | | 33 | 234,5 |
| Pourcentage de surface de feuilles attaquées | 1 jour après le le 3ème traitement | 1,2 | 7 |
| | 5 jours après le le 3ème traitement | 2,2 | 15 |
| | 4 jours après le le 4ème traitement | 2,7 | 30 |
| | 10 jours après le le 4ème traitement | 4,7 | 40 |

Exemple 3 :

On a traité deux séries de 4 parcelles de terrain de 10 m2 portant chacune une culture de tomate de la variété Marmande infestée par du mildiou (Phytophthora Infestans). Ces plants ont été traités 4 fois à 8 jours d'intervalle à l'aide de 2 bouillies fongicides définies ci-après et répandues à raison de 1000 l/ha.

Les bouillies utilisées ne différaient de celles de l'exemple 2 que par la dilution à l'eau qui était telle que la concentration en matière active était de 5 g/hl.

Les matières actives mises en oeuvre étaient celles utilisées à l'exemple 2.

Les résultats suivants ont été obtenus :

| | | COMPOSE A | COMPOSE N°39 |
|---|---|---|---|
| Nombre de folioles attaqués pour 10 plantes Mesure effectuée 1 jour après le 4ème traitement | | 23,3 | 371,3 |
| Pourcentage de surface de feuilles attaquées | 1 jour après le le 4ème traitement | 3,7 | 50 |
| | 9 jours après le le 4ème traitement | 10 | 91 |
| | 25 jours après le le 4ème traitement | 48 | 97,7 |
| Pourcentage de tomates (fruits) attaquées 16 jours après le le 4ème traitement | | 9,1 | 80,9 |

Exemple 4 :

On a traité 2 séries de 4 parcelles de terrain de 10 m2 portant chacune une culture de vigne (cépage GAMAY) infestée par du mildiou (Plasmopara viticola). Ces plants de vignes ont été traités 8 fois à environ 11 jours d'intervalle à l'aide de 2 bouillies fongicide définies ci-après et répandues à raison de 1000 l/ha.

Les bouillies utilisées ne différaient de celles de l'exemple 2 que par la dilution à l'eau qui étit telle que la concentration en matière active était de 22,5 g/hl.

Les résultats suivants ont été obtenus :

| | | COMPOSE A | COMPOSE N°39 |
|---|---|---|---|
| Pourcentage du nombre de feuilles attaquées | 7 jours après le le 6ème traitement | 10 | 66 |
| | 8 jours après le le 7ème traitement | 10 | 76 |
| | 30 jours après le le 8ème traitement | 12 | 80,5 |
| | 60 jours après le le 8ème traitement | 12 | 92 |

Exemple 5 : Essai "in vivo" contre mildiou de la tomate (Phytophtora infestans) sur plants de tomates. Associations.

Des plants de tomate (Lycopersicum esculentum), de variété Marmande, sont cultivées dans des godets. Lorsque ces plants sont âgés d'un mois (stade 5 à 6 feuilles, hauteur 12 à 15 cm), ils sont traités par pulvérisation au moyen d'une suspension ou solution aqueuse de la matière à tester, à la concentration désirée. Chaque plant de tomate reçoit environ 5 ml de la solution ou dispersion. Pour chaque concentration de matière active à tester, le traitement est effectué sur deux plants.

Cette suspension est obtenue en broyant mécaniquement 100 mg de matière active dans 100 ml d'une solution contenant 0,05 % de Tween 80 (condensat de monooléate de sorbitan et de 20 molécules d'oxyde d'éthylène). On a donc une suspension de produit fongicide de 1 g/l. Par dilutions successives dans de l'eau distillée, les concentrations désirées sont obtenues.

Des plants utilisés comme témoins sont traités par la même solution mais ne contenant pas de matière active.

Après séchage pendant 24 heures, on contamine chaque plant par pulvérisation au moyen d'une suspension aqueuse de spores de Phytophthora infestans, responsable du mildiou de la tomate, à raison d'environ 1 ml/plant (soit

environ $1.10^5$ spores par plant).

Après cette contamination, les plants de tomate sont placés dans des sacs plastiques individuels afin d'éviter des interférences entre les produits puis sont mis en incubation pendant deux jours à 15°C environ en atmosphère saturée d'humidité, puis pendant cinq jours à 17°C environ sous 70 % à 90 % d'humidité relative.

Sept jours après la contamination, on compare les résultats obtenus dans le cas des plants traités par la matière active à tester à ceux obtenus dans le cas des plants utilisés comme témoins.

OXYCHLORURE DE CUIVRE + COMPOSE DE L'EXEMPLE 1

| | concentration mg/l | % efficacité |
|---|---|---|
| Composé de l'exemple 1(A) | 8 | 100 |
| " | 4 | 92 |
| " | 2 | 58 |
| " | 1 | 0 |
| Oxychlorure de cuivre (B) | 4000 | 0 |
| " | 2000 | 0 |
| " | 1000 | 0 |
| " | 500 | 0 |

| | | |
|---|---|---|
| A + B | 8 + 4000 | 100 |
| | 8 + 2000 | 97 |
| | | |
| | 4 + 4000 | 99 |
| | 4 + 2000 | 99 |
| | 4 + 1000 | 78 |
| | 4 + 500 | 83 |
| | | |
| | 2 + 1000 | 49 |
| | 2 + 500 | 92 |
| | | |
| | 1 + 1000 | 63 |
| | 1 + 500 | 92 |

MANCOZEBE + COMPOSE DE L'EXEMPLE 1

| Produits | Concentrations mg/l | % |
|---|---|---|
| Composé de l'exemple 2(A) | 8 | 100 |
| | 4 | 92 |
| | 2 | 58 |
| | 1 | 0 |
| | | |
| Mancozebe (B) | 1000 | 100 |
| | 500 | 45 |
| | 250 | 28 |
| | 125 | 0 |
| | | |
| A + B | 8 + 500 | 100 |
| | 8 + 250 | 83 |
| | | |
| | 4 + 500 | 80 |
| | 4 + 250 | 93 |
| | 4 + 125 | 48 |
| | | |
| | 2 + 500 | 88 |
| | 2 + 250 | 56 |
| | 2 + 125 | 71 |
| | | |
| | 1 + 500 | 32 |
| | 1 + 250 | 76 |
| | 1 + 125 | 5 |

CAPTANE + COMPOSE DE L'EXEMPLE 1

| Produits | Concentrations mg/1 | % |
|---|---|---|
| Composé de l'exemple 1(A) | 8 | 100 |
| | 4 | 92 |
| | 2 | 58 |
| | 1 | 0 |
| | | |
| Captane (B) | 4000 | 0 |
| | 2000 | 0 |
| | 1000 | 0 |
| | 500 | 0 |
| | | |
| A + B | 8 + 4000 | 97 |
| | 8 + 2000 | 100 |
| | | |
| | 4 + 4000 | 96 |
| | 4 + 2000 | 92 |
| | 4 + 1000 | 90 |
| | 4 + 500 | 92 |
| | | |
| | 2 + 1000 | 85 |
| | 2 + 500 | 98 |
| | | |
| | 1 + 1000 | 86 |
| | 1 + 500 | 9 |

TABLEAU DE NOMENCLATURE

| | |
|---|---|
| Chlorothalonil | Tétrachloro-isophtalonitrile |
| Iprodione | 3-(3,5-dichlorophényl)-N-isopropyl-2,4-dioxo imidazolidine-1-carboxamide |
| Fenpropimorphe | (±)-cis-4-[3-(4-tert-butylphenyl)-2-methyl propyl]-2,6-diméthylmorpholine |
| Tridémorphe | 2,6-diméthyl-4-tridécylmorpholine |
| Fenpropidinel | [3-(p-terbutylphenyl)2-méthyl propyl] piperidine |
| Dinocap | 2-(1-méthylheptyl)-4,6-dinitrophényl crotonate |
| Dithianon | 5,10-dihydro-5,10-dioxonaphto [2,3-b]-1,4-dithia-anthraquinone |
| Manèbe | Ethylène bis (dithiocarbamate) de manganèse |
| Mancozèbe | Complexe de manèbe avec un sel de zinc |
| Zinèbe | Ethylène bis (dithiocarbamate) de zinc |
| Phoséthyl-Al | Tris-O-éthylphosphonate d'aluminium |
| Captane | N-(trichlorométhylthio) cyclohex-4-ene-1,2 dicarboximide |
| Carbendazime | Méthyl benzimidazol-2-yl carbamate |
| Captafol | N-(1,1,2,2-tétrachloroéthylthio) cyclohex-4-ene-1,2-dicarboximide |
| Cymoxanil | 2-cyano-N-[(éthylamino) carbonyl]-2-(méthoxyimino) acétamide |
| Nuarimol | α-(2-chlorophényl)-α-(4-fluoro -phényl)-5-pyrimidine méthanol |
| Diniconazol | 1-(2,4-dichlorophenyl)-4,4-dimethyl-2-(1,2,4-triazol-1-yl)-1-penten-3-ol. |

Ce dernier produit est décrit dans le brevet GB 2046260 déja mentionné.

| | |
|---|---|
| Cymoxanil | 1-(2-cyano-2-méthoxy iminoacétyl)-3-éthylurée. |
| Métalaxyl | N-(12,6-diméthylphényl phényl)-N-(2-méthoxy acétyl)-DL alaninate de méthyle. |
| Furalaxyl | N-(2,6-diméthyl phényl)-N-(2-furanyl carbonyl)-DL alaninate de méthyle. |
| Benalaxyl | N-phényl acétyl-N-2,6-xylyl-DL-alaninate de méthyle. |
| Oxadixyl | 2-méthoxy-N-(2-oxo-1,3-oxazolidin-3yl)acet-2',6' -xylidide. |
| Phosétyl-Al | (voir description). |

Les noms sont indiqués selon la nomenclature française mis à part le fait que les positions des substituants sont indiquées avant les substituants eux-mêmes.

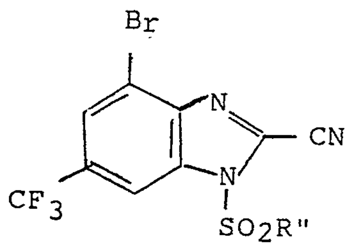

(I)

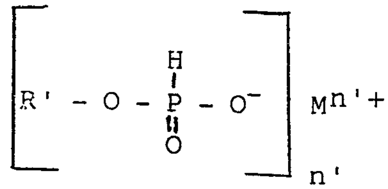

(II)

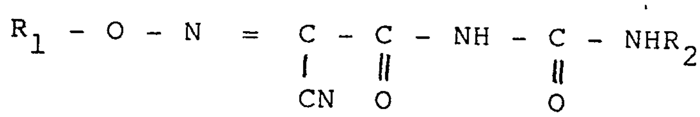

(III)

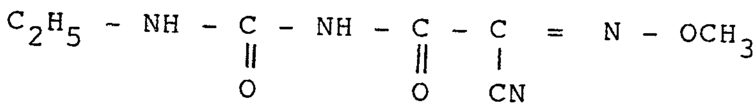

Cymoxanil

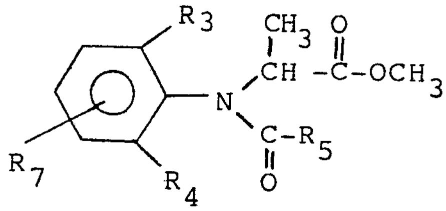

(IV)

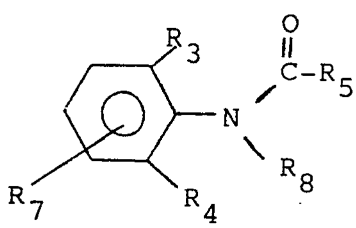

(V)

$R_8$ =

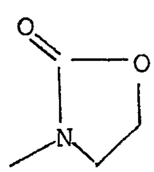

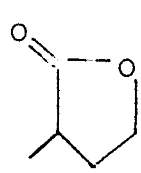

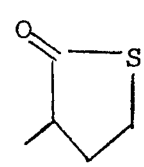

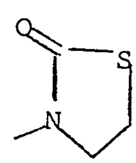

3-oxazolidinonyl 3-butyrolactonyl 3-thiobutyrolactonyl 3-thiooxazolidinonyl

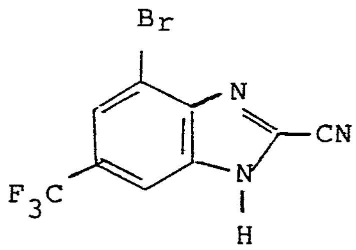

(VI)

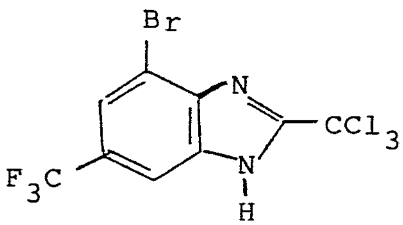

(VIII)

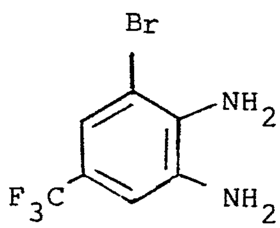

(IX)

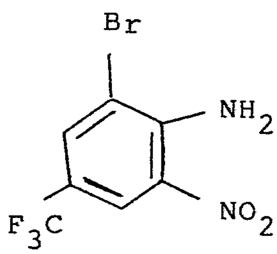

(X)

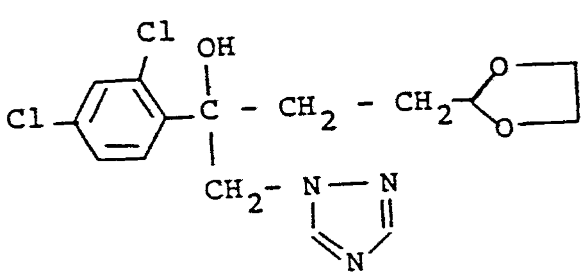

(XI)

REVENDICATIONS

1) Dérivé du cyano-2 benzimidazole caractérisé en ce qu'il a pour formule (I) indiquée dans la description dans laquelle R" représente un radical dialkylamino comportant de 2 à 4 atomes de carbone.

2) Dérivé selon la revendication 1, caractérisée en ce que R" représente le radical diméthylamino.

3) Composition antifongique caractérisée en ce qu'elle comprend comme matière active un composé selon l'une des revendications 1 ou 2.

4) Composition selon la revendication 3, caractérisée en ce que en plus de la matière active elle comprend un support inerte et/ou un agent tensioactif utilisable en agriculture.

5) Composition selon la revendication 4, caractérisée en ce qu'elle comprend de 0,5 à 95 % en poids, de matière active et de 0 à 20 %, en poids, d'agent tensioactif.

6) Procédé de préparation d'un composé selon la revendication 1,caractérisé en ce qu'il consiste à faire réagir le cyano-2 bromo-4 trifluorométhyl-6 benzimidazole de formule (VI) ou un sel de métal alcalin ou alcalino-terreux ou d'ammonium éventuellement substitué de ce composé, sur un hanogénure de formule (VII) :

$X - SO_2 R"$

dans laquelle R" a la même signification que dans la revendication 1 et X représente un atome d'halogène, de préférence l'atome de chlore.

7) Procédé selon la revendication 6, caractérisé en ce que la réaction entre le cyano-2 bromo-4 trifluorométhyl-6 benzimidazole et l'halogénure s'effectue dans un solvant aprotique polaire en présence d'un accepteur d'acide

8) Procédé selon la revendication 7, caractérisé en ce que la réaction entre le sel de métal alcalin ou alcalino-terreux ou d'ammonium éventuellement substitué

du benzimidazole et l'halogénure s'effectue dans un solvant aprotique polaire.

9) Utilisation des dérivés selon la revendication 1 ou 2 à titre de fongicide

10)Utilisation des dérivés selon la revendication 9 à titre de fongicide antimildiou notamment des solanacées, du tabac, de la vigne et des cucurbitacées.

11)Procédé de traitement des végétaux contre les champignons phytopathogènes, caractérisé en ce qu'il consiste à appliquer sur ces végétaux une quantité efficace d'un dérivé ou d'une composition composé selon l'une des revendications 1 à 5.

12)Procédé selon la revendication 11, caractérisé en ce que l'on applique 2 g/ha à 250 g/ha, de préférence 4 g/ha à 220 g/ha de dérivé selon la revendication 1 ou 2.

13)Association d'un ou plusieurs dérivés de formule (I) avec au moins un fongicide du groupe (II) constitué par les sous classes suivantes :

1. Les dérivés chlorés ou nitrés du benzène comme le quintozène ou le chlorothalonil,
2. Les dérivés dicarboximides et imides cycliques comme le captane, le folpel, le captafol, l'iprodione, la procymidone, la vinclozoline, le fluoroimide, le chlozolinate, le metomeclan,
3. Les dérivés comprenant un ou plusieurs hétérocycles comme les quinoléines (éthoxyquine), les morpholines (dodémorphe, tridémorphe, fenpropimorphe), les pipéridines (fenpropidine), les piperazines (triforine),
4. Les dérivés de l'acide dithiocarbamique comme le manèbe, le mancozèbe , le thirame et le metiram ou le zinèbe ou le propinèbe.
5. Les dérivés du phénol comme le dinocap ou le binapacryl,
6. Les dérivés des quinones comme le dithianon, le chloranil,
7. Les dérivés de l'acide carbamique et des benzimidazoles comme le carbendazime, le bénomyl, le thiophanate-méthyl,

thiabendazole, le fubéridazol.

8. Les dérivé soufrés comme le dazomet ou l'étridiazole ou le soufre, le fenaminosulf,

9. Les diazines ou triazines telles que le chinométhionate, le fénarimol, l'anilazine, le nuarimol, le bupirimat, l'éthylrimol, le pyrazophos, la diclomezine,

10. Les sulfamides telles que le dichlofluanide, le tolylfluanide,

11. Les guanidines telles que la doguadine,

12. Les triazoles tels que par exemple ceux décrits dans le brevet britannique n°2046260 dont le contenu est incorporé par référence comme le diniconazole ou d'autres triazoles connus, le propiconazole, le triadimefon, le triadimenol, le diclobutrazol, le bitertanol, le penconazol, l'hexaconazole, le flusilazole, le myclobutanil, le SAN 619F (Sandoz), l'ethyltrianol, le fluotrimazole, le flutriafol, les triazoles tels que ceux décrits dans la demande de brevet européen n°0151084, le triazole denommé : 2-(2,4-dichloro 1-phényl) 2-méthyl (1,2,4-triazol 1-yl) 5-trifluoroéthoxy tétrahydrofuranne ou ses formes salifiées.

13. Les imidazoles comme le prochloraz ou l'imazalil, le triflumizole,

14. Le cuivre ou les dérivés organiques ou inorganiques du cuivre comme le cuprosan.

15. Les organo stanniques comme le fentine acétate.

16. Les anilazines.

17. Les phosphites d'amines décrits dans le brevet belge n°890 114, dont le contenu est incorporé par référence.

18. Les dérivés de la pyridine tels que le buthiobate, le pyrifenox.

19. Les dérivés de l'amino-2 pyridine tels que le fluazinam.

20. Les dérivés de l'isoxazole et de l'isoxazolone tels que l'hymexazole et le drazoxolon.

21. Les dérivés des phényl-carbamates tels que le diethofencarb (isopropyl 3,4-diethoxyphenyl carbamate), le

methyl N-(3,5-dichlorophenyl) carbamate, et les autres produits actifs sur les souches de champignons résistantes aux benzimidazoles (par exemple : Botrytis cinerea).

14)Associations selon la revendication 13, caractérisé en ce que le groupe (II) est constitué par les fongicides suivants : chlorothalonil, iprodione, fentine-acétate, dinocap, manèbe, mancozèbe, zinèbe, captane, captafol, folpel, procymidone, le cuivre et les dérivés du cuivre tels que l'oxychlorure de cuivre ou quinoléate de cuivre.

15)Associations selon l'une des revendications 13 ou 14 caractérisé en ce qu'elles comprennent en plus un ou plusieurs fongicides choisis parmi les suivants :

a) l'acide phosphoreux ou ses sels alcalins ou les dérivés de l'acide phosphoreux de préférence les dérivés de formule (II) dans laquelle :

R' est un atome d'hydrogène ou de métal alcalin ou un radical alkyle contenant de 2 à 4 atomes de carbone ;

M est un atome d'hydrogène ou un atome d'un métal alcalin, alcalino terreux ou d'aluminium ;

n' est un nombre entier de 1 à 3 égal à la valence M.

De préférence, lorsque R' est un atome d'hydrogène ou de métal alcalin, M est un métal alcalin et n'=1.

De préférence, le dérivé est un alcoyl-phosphite métallique, de préférence le phosétyl-Al ou un dérivé alcalin de l'acide phosphoreux ou l'acide phosphoreux lui-même.

b) les acétamides tels que ceux décrits dans le brevet US n° 3,957,847 dont le contenu est incorporé par référence et de préférence répondant à la formule (III) dans laquelle : $R_1$, $R_2$ identiques ou différents représentent un groupement alkyle de $C_1$ à $C_{13}$ de préférence de $C_1$ à $C_4$ et de préférence le cymoxanil ($R_1 = CH_3$, $R_2 = C_2H_5$).

c) Les acylanilides tels que ceux décrits dans les brevets belges n° 827 671 et 827 419 ou européen n° 0026873 dont

le contenu est incorporé pr référence et de préférence :
1) ceux de formule (IV) dans laquelle :
$R_3$, $R_4$ identiques ou différents représentent un atome d'hydrogène ou un groupe alkyle de $C_1$ à $C_4$, de préférence le méthyle, ou alcoxy de $C_1$ à $C_4$, avantageusement $R_3 = R_4$ = méthyle.
$R_5$ représente un groupe $-CH_2-R_6$ dans lequel $R_6$ est un groupement alcoxy de $C_1$ à $C_4$, de préférence méthoxy, ou aryle de préférence phényle, un atome d'halogène de préférence le chlore, ou $R_5$ représente un cycloalkyle de $C_3$ à $C_6$ de préférence le cyclopropane, ou un hétérocycle en $C_5$ ou $C_6$, de préférence $C_5$, comportant un ou deux hétéroatomes de préférence le 2-furyle, le 5-isoxazolyle, le 3-oxazolyle.
$R_7$ est un atome d'hydrogène, d'halogène ou un radical alkyle de $C_2$ à $C_4$, de préférence méthyle et avantageusement, le métalaxyl, le furalaxyl, le bénalaxyl, le composé où $R_3=R_4=CH_3$, $R_7$ = H, $R_5$ = 5-isoxazolyle (LAB 149 202 F).
2) ou ceux de formule (V) dans laquelle :
$R_3$, $R_4$, $R_5$, $R_6$, $R_7$ ont la même signification que pour la formule (IV) avec les mêmes variantes préférées et $R_8$ est un cycle 3-oxazolidinonyl ou 3-butyrolactonyl ou le 3-thiobutyrolactonyl ou 3-thiooxazolidinonyl, avantageusement l'oxadixyl, le cyprofuram, l'ofurace, le CGA 68099 (RE 26745) dans lequel $R_3 = R_4$ = méthyle, $R_7$ = H, $R_5$ = $-CH_2OCH_3$, $R_8$ = 3-butyrolactonyl, le RE 26940 où $R_3 = R_4 = CH_3$, $R_7$ = H, $R_5 = CH_2OCH_3$, $R_8$ = 3-thiobutyrolactonyl, le M 12279 ou $R_3 = R_4 = CH_3$, $R_5 = -CH_2OCH_3$, $R_7$ = 3-Cl, $R_8$ = 3-oxazolidinonyl.

16) Associations selon l'une des revendications 13 à 15, caractérisé en ce que le rapport en poids du ou des composés du groupe (I) au ou aux composés du groupe (II)

est compris entre 1 : 10 000 et 100, de préférence entre 1/1000 et 10.

17)Associations selon l'une des revendications 13 à 16 pour une utilisation simultanée, séparée ou étalée dans le temps en traitement fongicides des végétaux, notamment fongicide antimildiou.

18)Utilisation des associations selon l'une des revendications 13 à 16 à titre de fongicide notamment antimildiou et de préférence des solanacées, du tabac, de la vigne, des cucurbitacées.

19)Composition fongicide notamment antimildiou caractérisée en ce qu'elle contient une association selon l'une des revendications 13 à 17 et au moins un support inerte acceptable en agriculture.

20)Composition selon la revendication 19, caractérisée en ce qu'elle contient 0,5 à 95 % de principe actif.

21)Procédé pour combattre les maladies fongiques des plantes, notamment le mildiou, caractérisé en ce qu'on applique sur ces plantes une quantité efficace d'une association selon l'une des revendications 13 à 17 ou d'une composition selon l'une des revendications 19 ou 20.

22)Procédé selon la revendication 21, caractérisé en ce qu'on applique le dérivé selon la revendication 1 ou une composition en résultant selon l'une des revendications 3 à 5 à raison de :

2 à 250 g/ha pour le dérivé de formule (I) et 100 à 3000 g/ha pour le ou les composés du groupe (II).

23)Procédé de préparation des associtions selon l'une des revendications 13 à 17, caractérisé en ce qu'on mélange le ou les composés du groupe (I) avec le ou les composés du groupe (II).

24)Composés de formule (VI) ou (VIII) et sels de métaux alcalins ou alcalino-terreux, ou d'ammonium éventuellement substitué de ce composé.

25)Dérivés de l'aniline, de formule IX ou X ou un sel de ces composés formé avec un acide fort, ou la bromo-2 trifluorométhyl-4 nitro-6 aniline.

26)Composés selon la revendication 25, caractérisés en ce que le sel formé avec un acide fort est le monochlorhydrate de diamino-1,2 bromo-3 trifluorométhyl-5 benzène.